# EUROPEAN PATENT SPECIFICATION

(11) **EP 2 746 548 B1**
(45) Date of publication and mention of the grant of the patent: **15.03.2017**
(21) Application number: 12199278.8
(22) Date of filing: 21.12.2012
(51) Int. Cl.: F01N 3/20

(54) **Method and system for purifying the exhaust gases of a combustion engine.**
Verfahren und System zur Reinigung der Abgase eines Verbrennungsmotors
Procédé et système pour purifier les gaz d'échappement d'un moteur à combustion

(43) Date of publication of application: 25.06.2014
(73) Proprietor: Inergy Automotive Systems Research (Société Anonyme), 1120 Bruxelles (BE)
(72) Inventor: Dougnier, François, 3190 Boortmeerbeek (BE); Van Schaftingen, Jules Joseph, 1300 Wavre (BE); Madoux, Dominique, 7611 Rumes (BE); Wouters, Paul, 1120 Bruxelles (BE)
(74) Representative: Remy, Vincent Noel Paul

(56) References cited:
- WO-A1-98/42623
- WO-A1-2004/042207
- DE-A1- 4 425 420
- DE-A1-102008 042 735
- JP-A- 2005 273 509

## Description

The present application relates to a method and a system for purifying the exhaust gases of a combustion engine by injecting exclusively ammonia gas, and more particularly ammonia gas that is released from one or several solid absorbing matrices where it is stored by sorption.

Legislation on vehicle and truck emissions stipulates, amongst other things, a reduction in the release of nitrogen oxides NOₓ into the atmosphere. One known way to achieve this objective is to use the SCR (Selective Catalytic Reduction) process which enables the reduction of nitrogen oxides by injection of a reducing agent, generally ammonia, into the exhaust line. This ammonia may be obtained by using different techniques. One known technique is based on the use of a solid absorbing matrix where the ammonia is trapped by sorption. Generally, the solid absorbing matrix is stored in a container (or tank) (called hereafter matrix storage container) mounted on the vehicle. According to this known technique, the ammonia is released by heating the solid absorbing matrix, and then the released ammonia is injected into the exhaust line.

This known technique offers high performance since it allows to send pure NH₃ in the exhaust gases of the vehicle.

However, the main disadvantage of this known technique is the complexity of the refilling procedure (i.e. the solid absorbing matrix regeneration). Indeed, the actual refilling procedure consists of connecting the matrix storage container to an external ammonia source, typically a highly pressurized cylinder. In this procedure, the matrix storage container has to be dismounted from the vehicle for ammonia loading. Moreover, sending a high pressure of ammonia in the solid matrix results in a huge heat generation due to exothermal sorption reaction (tens of thousands J/mol NH₃, as a likely order of magnitude), hence the need of a cooling unit as heat sink. High temperature can damage parts of the system and induce long regeneration time.
It is also known from document WO2004/042207A a SCR method wherein ammonia gas is released from a solid absorbing matrix, and wherein the solid absorbing matrix is regenerated on board the vehicle, by generating a refilling ammonia gas by decomposing a ammonia precursor, and by directing the refilling ammonia gas to the solid absorbing matrix where it is stored thereon.

In view of the above-mentioned disadvantage, there exists a need for an improved method for the regeneration of the solid absorbing matrix.

An object of the present invention is to solve this above-mentioned problem by proposing an SCR method for_purifying the exhaust gases of an internal combustion engine of a vehicle, according to which ammonia gas is exclusively metered in the exhaust gases, the method comprising a step of releasing ammonia gas from at least one solid absorbing matrix where it is stored by sorption and a step of metering the released ammonia gas in the exhaust gases. According to one aspect of the present invention, the method comprises a step of regenerating the solid absorbing matrix that consists in:
- generating a refilling ammonia gas by chemically decomposing a ammonia precursor in a biochemical decomposition unit mounted on board the vehicle and storing at least one protein component adapted to decompose said ammonia precursor;
- separating water from the refilling ammonia gas by means of a liquid-vapour separator unit;
- directing the refilling ammonia gas to the solid absorbing matrix where it is stored thereon.

Thus, it is proposed an in situ regeneration procedure. In other words, the regeneration of the solid absorbing matrix takes place on board the vehicle. More precisely, the regeneration procedure according to the invention is based on the decomposition of an ammonia precursor. Such decomposition is obtained by using a biochemical decomposition unit mounted on board the vehicle. The biochemical decomposition unit according to the invention stores one or several protein component(s) that catalyze a chemical reaction. More precisely, the protein component(s) is(are) adapted to catalyze the hydrolysis (i.e. decomposition) of the ammonia precursor to ammonia. This decomposition results in the generation of a refilling ammonia gas. The refilling ammonia gas is then directed (i.e. transmitted) to the solid absorbing matrix where it is stored thereon. According to the invention, no external ammonia source is used and no disassembly manual operations are needed for the regeneration of the solid absorbing matrix(ces). Thus, the regeneration procedure according to the invention is simple, faster and safer.

In a preferred embodiment, a predetermined amount of ammonia precursor is stored on board the vehicle. For example, during vehicle (engine) operation, it is calculated a desired amount of ammonia precursor to be injected into the biochemical decomposition unit. Such calculation can be made as a function of information relative to the amount of ammonia gas that has been injected into the exhaust line. In a particular embodiment, such information may derive from data provided by a temperature sensor, a pressure sensor or a flow meter, or any combination of these sensors. In another particular embodiment, such information may derive from data provided by a device configured to measure the concentration of ammonia stored in the solid absorbing matrix. In another particular embodiment, this information may be derived from an estimation of the consumption of ammonia.

Advantageously, the protein component (stored in the biochemical decomposition unit) comprises at least one enzyme. In particular, thermophile-type enzymes are well suited. In a preferred embodiment, the biochemical decomposition unit can store urease. Urease can be stored in any suitable manner. For example, in a first embodiment urease can be immobilized in different layers of resin. In a second embodiment urease can be fixed on membranes.

The SCR method according to the present invention is aiming at injecting exclusively ammonia gas in the exhaust gases. In other words, no ammonia precursor is injected in the exhaust gases.

In a particular embodiment, the ammonia precursor is an aqueous urea solution.

The terms "urea solution" are understood to mean any, generally aqueous, solution containing urea. The invention gives good results with eutectic water/urea solutions for which there is a quality standard: for example, according to the standard ISO 22241, in the case of the AdBlue^{®} solution (commercial solution of urea), the urea content is between 31.8 % and 33.2 % (by weight) (i.e. 32.5 +/- 0.7 wt%) hence an available amount of ammonia between 18.0 % and 18.8 %. The invention may also be applied to the urea/ammonium formate mixtures, also in aqueous solution, sold under the trade name Denoxium™ and of which one of the compositions (Denoxium-30) contains an equivalent amount of ammonia to that of the AdBlue^{®} solution. The latter have the advantage of only freezing from -30°C onwards (as opposed to -11°C), but have the disadvantages of corrosion problems linked to the possible release of formic acid. The invention can also apply to guanidinium formate. The present invention is particularly advantageous in the context of eutectic water/urea solutions, which are widely available in gas stations.

According to the invention, no urea solution is injected in the exhaust gases. There is no line (or conduit) for transporting the urea solution up to the exhaust line and there is no metering device for injecting the urea solution in the exhaust gases. According to the invention, the urea solution is exclusively transported to the biochemical decomposition unit, where it enters into a chemical reaction with the enzyme so as to generate the refilling ammonia gas.

In a particular embodiment, the urea solution can be partially stored in a chamber located within the biochemical decomposition unit, before it is chemically decomposed.

In another particular embodiment, the solid absorbing matrix is stored in a first tank and the ammonia precursor is stored in a second tank. Advantageously, the second tank (storing the ammonia precursor) is connected in a communicating manner to said biochemical decomposition unit, and said biochemical decomposition unit is connected in a communicating manner to the first tank (storing the solid absorbing matrix). In a first embodiment, the first tank and the second tank can be separate storage tanks. In a second embodiment, the first tank and the second tank can be two separate chambers of a same container.

It should be noted that it exists well known refilling standards and systems for ammonia precursor, in particular for the AdBlue^{®} solution (commercial solution of urea). The refilling of the storage tank of the ammonia precursor is trivial. For example, this can be achieved by using available standard-designed nozzle and/or bottles with dedicated interfaces.

In a particular embodiment, the biochemical decomposition unit can be located below the storage tank of the ammonia precursor. In this particular embodiment, a stream (i.e. part) of the ammonia precursor can be transported towards the biochemical decomposition unit by gravity.

According to the invention, if the ammonia precursor generates water by thermal decomposition, this water is separated from the ammonia, collected and preferably prevented from being stored on the solid absorbing matrix. Separation of the water from the ammonia, and generally from the eventual other thermal decomposition products (generally gases like CO₂), is made using a liquid-vapour separator unit. For example, the liquid-vapour separator unit can comprise (or be) a condenser or one or several membranes like disclosed in US patent 4758250 for instance, which is a polymeric membrane. The condenser may be a specific one comprising a specific shaped tube having different parts at different temperatures (as described in example 2 below); a phase change material, or any other means for cooling and condensing the gases. Alternatively, the condenser may be part of a device already onboard the vehicle, for instance: part of the vehicle air conditioning system.

The water collected may be vaporised in the exhaust gases and/or at least part of it can be stored for instance to be available for dissolving excess ammonia that would pressurize unduly the storage tank of the solid absorbing matrix (see embodiment with pressure relief valve described below).

The ammonia is metered using a gas line which may comprise a non return valve close to the metering point.

The method according to the invention uses two separate storage tanks: one for the ammonia precursor and one for the solid absorbing matrix which stores ammonia by sorption. As described in patent application WO 2006/012903, metal ammine salts (preferably alkaline earth metal chlorides) can be used as solid storage media for ammonia.

Advantageously, the biochemical decomposition unit is equipped with a heater. Such heater can provide the optimum temperature for the desired activity of the enzyme or protein. For example, the heater can be configured to maintain within the biochemical decomposition unit a temperature range between 30°C and 60°C.

More generally, the heater is a chamber whose temperature is controlled within predetermined ranges; in case the predetermined range falls below the temperature of the environment, cooling means will also be made available within the heater. In other words, the heater can either be controlled so as to rise up the temperature within the chamber or controlled so as to cool down the temperature within the chamber.

In a particular embodiment, the heater is configured to work within at least one predetermined temperature range corresponding to the activation of the protein component when conversion is needed, and within at least another predetermined temperature range corresponding to the preservation of the protein component, so as to extend its lifetime.

In a particular embodiment of the invention, the heater can comprise resistive heating elements. These resistive heating elements may be metallic heating filaments (wires), flexible heaters, (that is to say heaters comprising one or more resistive track(s) affixed to a film or placed between two films (that is to say two substantially flat supports, the material and thickness of which are such that they are flexible)) or any other type of resistive elements that have a shape, size and flexibility suitable for being inserted into and/or wound around the components of the SCR system. PTC (Positive Temperature Coefficient) elements are more particularly suitable for heating.

In another particular embodiment of the invention, the heater uses the dissipated heat of the engine (for instance, a flow of the liquid engine cooling system) and/or exhaust line (gases) for heating the biochemical decomposition unit.

According to the invention, after generating the refilling ammonia gas, it can be compressed by means of a gas pressurisation unit. The function of this gas pressurisation unit is to compress the refilling ammonia gas to a suitable pressure for absorption by the solid absorbing matrix.

When heating the solid absorbing matrix to release ammonia, it can be that too high ammonia pressure build up occurs inside the system, due to thermal inertia or to a potential failure of the heating power regulation. In order to release the pressure above a given set-point, the excess of gaseous ammonia is preferably released by a safety valve and either directly returned to the ammonia precursor tank (preferred embodiment in the case of a solid ammonia precursor), or first dissolved in an adequate amount of water, for instance coming from the evaporation of the precursor solution the case being, and stored on purpose, and at a composition involving an amount of available ammonia identical to the one of the precursor solution (preferred embodiment in the case of urea precursor solutions). In another preferred embodiment, the excess of ammonia released can merely be dissolved in water and the ammonia solution so obtained can be used later on for thermal ammonia generation and storage on the solid absorbing matrix.

The present invention also concerns a system for applying the SCR method as described above, said system comprising:
- a first tank mounted on board a vehicle and storing at least one solid absorbing matrix where ammonia is stored by sorption,
- means for metering ammonia gas released from the solid absorbing matrix, in the exhaust gases,
- means for directing a stream of ammonia precursor solution to a biochemical decomposition unit mounted on board the vehicle and storing at least one protein component adapted to decompose said stream to generate a refilling ammonia gas;
- a liquid-vapour separator unit for separating water from the refilling ammonia gas;
- means for directing the refilling ammonia gas to the solid absorbing matrix stored in the first tank.

Preferably, the storage tank for the solid absorbing matrix comprises or is connected to a pressure release valve as described above.

The means for directing the stream of ammonia precursor solution to the biochemical decomposition unit generally comprise a pipe, a valve and eventually a pump, although if the biochemical decomposition unit is located below the storage tank of the ammonia precursor solution, the stream can merely be generated by gravity.

The means for separating the water from the ammonia may be a condenser and/or a membrane as set forth above.

The means for directing the refilling ammonia gas to the storage tank of the solid storage absorbing matrix may be a simple tube (pipe) and said refilling ammonia gas may be mixed with other gaseous decomposition product(s) like CO₂ for instance.

In one embodiment, the system of the invention also comprises a pressure relief valve enabling to release pressure above a given set point in the storage tank of the solid absorbing matrix. Preferably, it also comprises means for dissolving the gases so released into a given amount of water and means for returning the so obtained solution to the storage tank of an ammonia precursor solution.

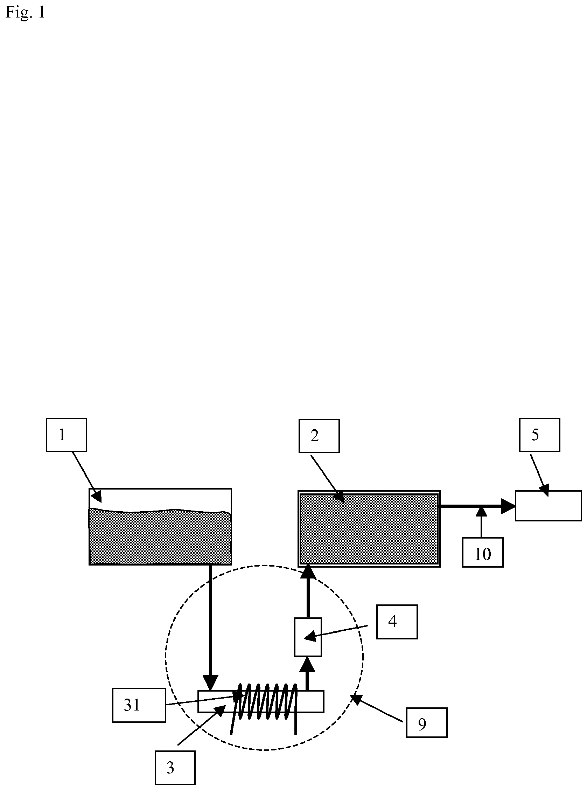

The present invention is illustrated in a non limitative way by the examples below relying on figures 1 to 5 attached. In these figures, identical or similar devices bear identical reference numbers.

### Example 1

Figure 1 is a schematic view of a SCR system according to a particular embodiment of the present invention.

As illustrated in Figure 1, a liquid solution of an ammonia precursor is stored in a tank [1] and a solid absorbing matrix is stored in a tank [2]. The tank [1] and the tank [2] are connected together in a communicating manner via a communication line [9]. The communication line [9] comprises a biochemical decomposition unit [3] and a condenser [4].

The SCR system of the invention is designed to exclusively inject ammonia gas (NH₃) in the exhaust gases. Thus, the SCR system of the invention is simple and efficient, since only pure NH₃ is sent in the exhaust gases (no liquid solution or solid compound is injected in the exhaust gases).

According to one aspect of the invention, the biochemical decomposition unit [3] receives a stream (i.e. part) of the ammonia precursor. The biochemical decomposition unit [3] contains an enzyme (for example, urease) that catalyzes the hydrolysis (i.e. decomposition) of the ammonia precursor to ammonia. Thus, the biochemical decomposition unit [3] generates the refilling ammonia gas for the regeneration of the solid absorbing matrix.

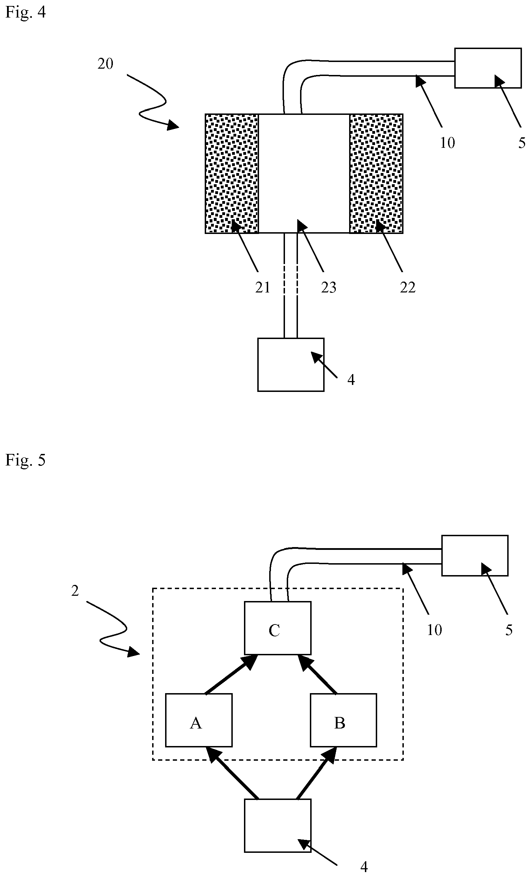

Figure 4 is a schematic view of the tank [2] according to a particular embodiment of the present invention. As illustrated in this example, the tank [2] comprises one cell [20]. The cell [20] comprises two chambers [21] and [22], each containing a solid absorbing matrix. The chambers can contain similar or distinct type of solid absorbing matrix. The chambers [21] and [22] are separated by a gas flow channel [23]. The ammonia gas released from the solid absorbing matrices (contained in the chambers [21] and [22]) and (eventually) the refilling ammonia gas (generated by the biochemical decomposition unit) can flow through the channel [23] towards the condenser [4]. Of course, in another embodiment the cell [20] can comprise one or more than two chamber(s).

According to another particular embodiment of the present invention, the tank [2] can comprise a plurality of cells connected in series and/or in parallel.

Figure 5 is a schematic view of the tank [2] according to another particular embodiment of the present invention. As illustrated in this example, the tank [2] comprises three cells (A, B, C) containing, for example, solid materials showing different ammonia sorption properties. The tank [2] is based on a two-stage unit. The first stage comprises the cells A and B, and the second stage comprises the cell C. For example, the cells A and B are filled with magnesium chloride and the cell C is filled with calcium chloride or barium chloride. One magnesium chloride-filled cell (for example, cell A) is used for the absorption of ammonia generated by the biochemical decomposition unit [3] while the second one (for example, cell B), previously saturated with ammonia (coming from the biochemical decomposition unit [3]), is used to provide ammonia gas which is further absorbed in the cell C. When the cell A is ammonia saturated and the cell B is empty, the roles of cells A and B are reversed. This two-stage unit combines the enhanced absorption properties of one matrix material (for example, magnesium chloride) for ammonia capture, and the advantage of the desorption properties of a second matrix material (for example, calcium chloride or barium chloride) to make ammonia readily available for the selective catalytic reduction (i.e. purification of the exhaust gases).

As illustrated in figure 1, the tank [2] is connected to the exhaust pipe [5] via an injection line [10] configured to transport exclusively ammonia gas. According to one aspect of the invention, ammonia gas (NH₃) released from the solid absorbing matrix flows through the injection line [10] and is metered in the exhaust pipe [5].

According to another aspect of the invention, no liquid solution is injected in the exhaust pipe [5]. As illustrated in figure 1, there is no line (or pipe) for transporting the liquid solution stored in tank [1] up to the exhaust pipe [5]. The liquid solution is exclusively transported to the biochemical decomposition unit [3], where it is chemically decomposed to generate the refilling ammonia gas for the solid absorbing matrix stored in tank [2].

The liquid solution stored in tank [1] is preferably a 32.5% urea solution commercially available under the brand name Adblue^{®}, but other soluble ammonia compounds (like ammonium carbamate or guanidinium formate) are also suitable. A stream (i.e. part) of the solution enters inside the biochemical decomposition unit [3], where water evaporation and urea decomposition occur. The water is further separated in the condenser [4], and the remaining gaseous flow (i.e. the refilling ammonia gas) goes through the tank [2] where ammonia is trapped on the solid absorbing matrix.

In a particular embodiment, for example when the solid absorbing matrix is saturated with ammonia, a stream (i.e. part) or all of the refilling ammonia gas can flow through the injection line [10] and can be metered in the exhaust pipe [5]. In this particular embodiment, the stream or all of the refilling ammonia gas can flow through the solid absorbing matrix, i.e. the stream or all of the refilling ammonia gas is not trapped on the solid absorbing matrix.

As illustrated in figure 1, the biochemical decomposition unit [3] is equipped with a heater [31]. The heater [31] can provide inside the biochemical decomposition unit [3] the optimum temperature for the desired activity of the enzyme. For example, the heat source of the heater [31] can be derived from a hot part of the vehicle, and is preferably a section of the exhaust line. Alternatively, the heater can also be electrical. For example, the temperature range is 30°C - 60°C.

In a particular embodiment, the refilling ammonia gas generated by the biochemical decomposition unit can be compressed to a suitable pressure before absorption by the solid absorbing matrix. To this aim, a gas pressurisation unit (not shown) can be mounted between the biochemical decomposition unit [3] and the condenser [4]. In a particular embodiment, the gas pressurisation unit can comprise a pump. In another embodiment, the gas pressurisation unit can comprise a piston system. In yet another embodiment, the gas pressurisation unit can comprise a controllable valve system.

As regards the content of the tank [2], any material showing ammonia sorption properties is convenient; however, a matrix containing alkaline earth metal chloride is particularly adapted. The excess of carbon dioxide is either trapped in the condensed water or released in the exhaust pipe [5], when the pressure inside the tank [2] reaches a pre-set level. In vehicle operation, ammonia is desorbed from the solid which is stored in the tank [2], and carried to the exhaust line [5], upstream of the SCR catalyst. The condensed water can be further vaporized in the exhaust line of the vehicle.

In a particular embodiment, an ammonia adsorption loop can also be used, for example in the form of a convection system with a carrier gas used for ammonia depletion of the biochemical decomposition unit [3], and transfer of the ammonia gas to the solid absorbing matrix. After absorption, the ammonia-free carrier gas is available to be enriched with ammonia by flowing again through the decomposition unit [3].

### Example 2

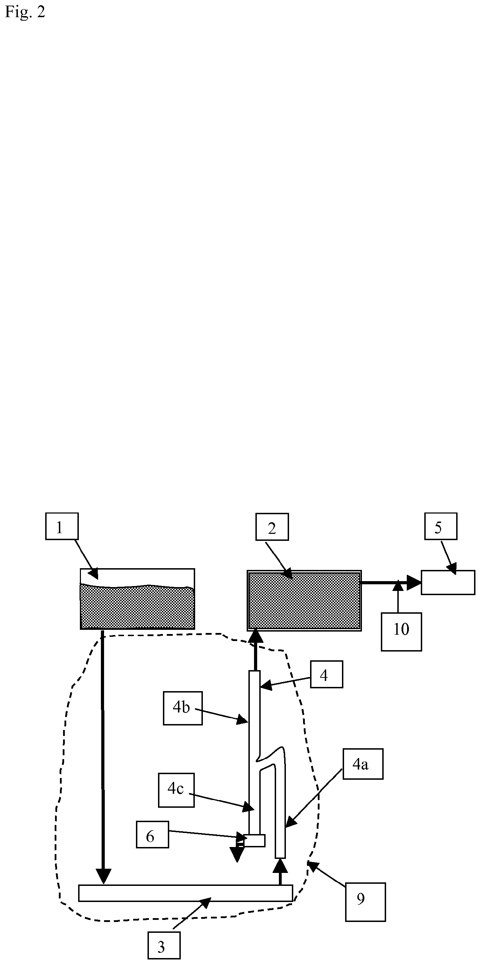

This example, relying on Figure 2 attached, illustrates the case in which the condenser [4] is made of a shaped tube. The tank [1] is filled with a urea solution. The gas flow resulting from the water evaporation and the urea decomposition goes through the inlet part [4a] of the condenser. Water vapors are condensed in part [4b] of the device, having a temperature lower than part [4a]. Liquid water is further collected in part [4c], and is removed by opening the valve [6]. Ammonia vapor goes to tank [2] through the outlet of the part [4b] of the condenser.

### Example 3

In this example, the condenser of example 1 is removed and the water/gas separation is made effective by using a membrane or a series of membranes at the outlet of the biochemical decomposition unit [3].

### Example 4

In this case, tank [1] is filled with a solid state ammonia precursor, for example: urea or ammonium carbamate, in the form of powder, pellets or flakes. A stream (part) of the solid is drawn to the biochemical decomposition unit [3] where ammonia is generated, and further trapped in a solid material in the tank [2]. No separator device (water condenser, for example) is needed in this example.

### Example 5

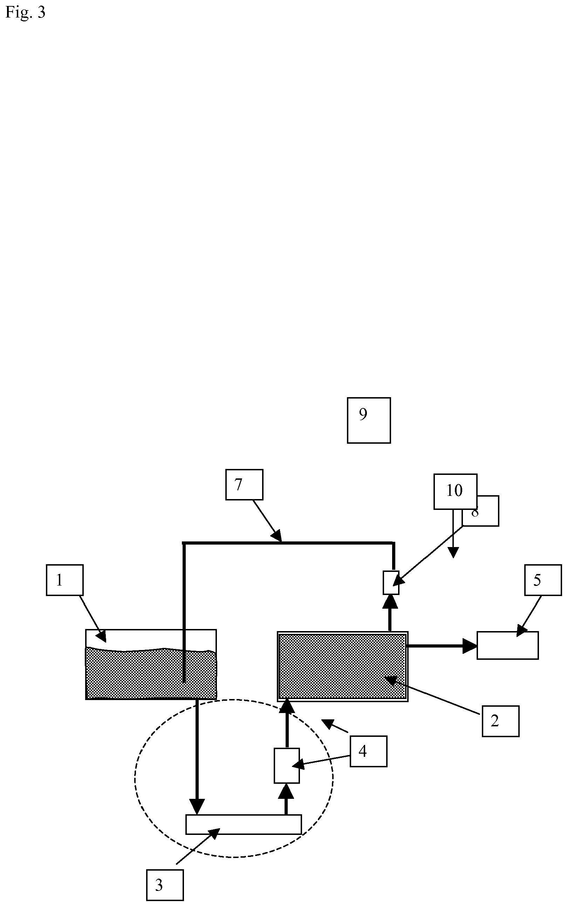

In this example, relying on Figure 3, a pressure safety function of tank [2] is described in this example. When pressure build up inside the tank [2] is higher than a set value, the pressure valve [8] is open, and the ammonia gas flows to the tank [1], through the line [7]. Ammonia is further dissolved in the solution which is stored in the tank [1].

In a preferred embodiment, which is not illustrated in Figure 3, line [7] does not return ammonia directly to tank [1] but instead, it conveys it first to a chamber/tank where it is dissolved in an appropriate amount of water so as to reach the right composition (preferably having the same amount of available ammonia as the solution in tank [1] and the urea solution so obtained is then sent to tank [1].

This can be done for instance by storing a given amount of water in the chamber, by deducing the amount of ammonia released from the pressure difference since the beginning of the pressure release and by returning the solution to the tank when the right ammonia concentration is reached.

## Claims

1. SCR or Selective Catalytic Reduction method for purifying the exhaust gases of an internal combustion engine of a vehicle, according to which ammonia gas is exclusively metered in the exhaust gases, the method comprising a step of releasing ammonia gas from at least one solid absorbing matrix where it is stored by sorption and a step of metering the released ammonia gas in the exhaust gases, wherein the method comprises a step of regenerating the solid absorbing matrix that consists in:
- generating a refilling ammonia gas by chemically decomposing a ammonia precursor in a biochemical decomposition unit (3) mounted on board the vehicle and storing at least one protein component adapted to decompose said ammonia precursor;
- separating water from the refilling ammonia gas by means of a liquid-vapour separator unit;
- directing the refilling ammonia gas to the solid absorbing matrix where it is stored thereon.

2. SCR method according to claim 1, wherein said protein component comprises at least one enzyme.

3. SCR method according to claim 2, wherein said enzyme is urease.

4. SCR method according to any one of claims 1 to 3, wherein said biochemical decomposition unit is equipped with a heater.

5. SCR method according to any one of claims 1 to 4, wherein it comprises a step of compressing the refilling ammonia gas by means of a gas pressurisation unit, the step of compressing being performed before the step of directing the refilling ammonia gas to the solid absorbing matrix.

6. SCR method according to the any one of claims 1 to 5, wherein a stream of the refilling ammonia gas is metered in the exhaust gases.

7. SCR method according to any one of claims 1 to 6, wherein the solid absorbing matrix is stored in a first tank (2) and the ammonia precursor is stored in a second tank (1), and wherein the second tank (1) is connected in a communicating manner to said biochemical decomposition unit (3), and said biochemical decomposition unit (3) is connected in a communicating manner to the first tank (2).

8. SCR method according to any one of claims 1 to 7, wherein the solid absorbing matrix comprises a plurality of cells containing material(s) adapted to store ammonia by sorption.

9. SCR method according to any one of claims 1 to 8, wherein the ammonia precursor is a solid compound.

10. SCR method according to any one of claims 1 to 8, wherein the ammonia precursor is an aqueous urea solution.

11. SCR method according to any one of claims 1 to 10, wherein the first tank (2) in which the solid absorbing matrix is stored is connected to a safety valve (8) configured to release the excess of gaseous ammonia in order to release the pressure above a given set-point.

12. System for applying an SCR method according to any one of the preceding claims, said system comprising:
- a first tank (2) mounted on board a vehicle and storing at least one solid absorbing matrix where ammonia is stored by sorption,
- means for metering ammonia gas released from the solid absorbing matrix, in the exhaust gases, **characterized in that** the system further comprises
- means for directing a stream of ammonia precursor solution to a biochemical decomposition unit (3) mounted on board the vehicle and storing at least one protein component adapted to decompose said stream to generate a refilling ammonia gas ;
- a liquid-vapour separator unit (4) for separating water from the refilling ammonia gas; means for directing the refilling ammonia gas to the solid absorbing matrix stored in the first tank (2).

13. System according to the preceding claim, wherein said protein component comprises at least one enzyme.

14. System according to the preceding claim, wherein said enzyme is urease.

## Patentansprüche

1. SCR-Verfahren oder Verfahren zur selektiven katalytischen Reduktion zum Reinigen des Abgases eines Verbrennungskraftmotors eines Fahrzeugs, wobei gemäß dem Verfahren Ammoniakgas ausschließlich in den Abgasen gemessen wird, das Verfahren umfassend einen Schritt des Freisetzens von Ammoniakgas von wenigstens einer festen absorbierenden Matrix, in welcher es gespeichert ist, durch Sorption, und einen Schritt des Messens des freigesetzten Ammoniakgases in den Abgasen, wobei das Verfahren einen Schritt des Regenerierens der festen absorbierenden Matrix umfasst, wobei dieser Schritt enthält:
- Generieren eines auffüllenden Ammoniakgases durch chemisches Zersetzen eines Vorprodukts von Ammoniak in einer Einheit (3) zur biochemischen Zersetzung, wobei die Einheit an Bord des Fahrzeugs montiert ist, und Speichern wenigstens einer Komponente eines Proteins, wobei die Komponente dazu eingerichtet ist, das genannte Vorprodukt von Ammoniak zu zersetzen;
- Trennen von Wasser von dem auffüllenden Ammoniakgas mittels einer Einheit zum Trennen von Flüssigkeit und Dampf;
- Leiten des auffüllenden Ammoniakgases zu der festen absorbierenden Matrix, wo es auf ihr gespeichert wird.

2. SCR-Verfahren nach Anspruch 1, wobei die genannte Komponente des Proteins wenigstens ein Enzym umfasst.

3. SCR-Verfahren nach Anspruch 2, wobei das genannte Enzym Urease ist.

4. SCR-Verfahren nach einem beliebigen der Ansprüche 1 bis 3, wobei die genannte Einheit zur biochemischen Zersetzung mit einer Heizung ausgestattet ist.

5. SCR-Verfahren nach einem beliebigen der Ansprüche 1 bis 4, wobei es einen Schritt des Verdichtens des auffüllenden Ammoniakgases mittels einer Einheit zum Verdichten von Gas umfasst, wobei der Schritt des Verdichtens vor dem Schritt der Leitung des auffüllenden Ammoniakgases zu der festen absorbierenden Matrix durchgeführt wird.

6. SCR-Verfahren nach einem beliebigen der Ansprüche 1 bis 5, wobei ein Strom des auffüllenden Ammoniakgases in den Abgasen gemessen wird.

7. SCR-Verfahren nach einem beliebigen der Ansprüche 1 bis 6, wobei die feste absorbierende Matrix in einem ersten Tank (2) gespeichert ist, und wobei das Vorprodukt von Ammoniak in einem zweiten Tank (1) gespeichert ist, und wobei der zweite Tank (1) in einer kommunizierenden Weise mit der genannten Einheit (3) zur biochemischen Zersetzung verbunden ist, und wobei die genannte Einheit (3) zur biochemischen Zersetzung in einer kommunizierenden Weise mit dem ersten Tank (2) verbunden ist.

8. SCR-Verfahren nach einem beliebigen der Ansprüche 1 bis 7, wobei die feste absorbierende Matrix eine Mehrzahl an Zellen umfasst, wobei die Zellen Material(ien) beinhalten, welche dazu eingerichtet sind, Ammoniak durch Sorption zu speichern.

9. SCR-Verfahren nach einem beliebigen der Ansprüche 1 bis 8, wobei das Vorprodukt von Ammoniak eine feste Verbindung ist.

10. SCR-Verfahren nach einem beliebigen der Ansprüche 1 bis 8, wobei das Vorprodukt von Ammoniak eine wässrige Harnstoff-Lösung ist.

11. SCR-Verfahren nach einem beliebigen der Ansprüche 1 bis 10, wobei der erste Tank (2), in welchem die feste absorbierende Matrix gespeichert ist, mit einem Sicherheitsventil (8) verbunden ist, wobei das Sicherheitsventil (8) dazu eingerichtet ist, den Überschuss an gasförmigen Ammoniak freizusetzen, um den Druck oberhalb eines gegebenen Sollwertes freizusetzen.

12. System zur Anwendung eines SCR-Verfahrens nach einem beliebigen der vorstehenden Ansprüche, wobei das genannte System umfasst:
- einen ersten Tank (2), welcher an Bord eines Fahrzeugs montiert ist und welcher wenigstens eine feste absorbierende Matrix speichert, wobei Ammoniak durch Sorption gespeichert wird,
- Mittel zum Messen von Ammoniakgas, welches von der festen absorbierenden Matrix freigesetzt worden ist, in den Abgasen, **dadurch gekennzeichnet, dass** das System weiterhin umfasst
- Mittel zum Leiten eines Stroms einer Lösung mit einem Vorprodukt von Ammoniak zu einer Einheit (3) zum biochemischen Zersetzen, wobei die Einheit (3) an Bord des Fahrzeugs montiert ist und wenigstens eine Komponente eines Proteins speichert, wobei die Komponente dazu eingerichtet ist, den genannten Strom zu zersetzen, um ein auffüllendes Ammoniakgas zu generieren;
- eine Einheit (4) zum Trennen von Flüssigkeit und Dampf, wobei die Einheit dazu bestimmt ist, Wasser von dem auffüllenden Ammoniakgas zu trennen; Mittel zum Leiten des auffüllenden Ammoniakgases zu der festen absorbierenden Matrix, welche in dem ersten Tank (2) gespeichert ist.

13. System nach dem vorstehenden Anspruch, wobei die genannte Komponente des Proteins wenigstens ein Enzym umfasst.

14. System nach dem vorstehenden Anspruch, wobei das genannte Enzym Urease ist.

## Revendications

1. Procédé SCR ou de réduction catalytique sélective pour purifier les gaz d'échappement d'un moteur à combustion interne d'un véhicule, selon lequel le gaz d'ammoniac est exclusivement dosé dans les gaz d'échappement, le procédé comprenant une étape de libération de gaz d'ammoniac à partir d'au moins une matrice absorbante solide où il est stocké par sorption et une étape de dosage du gaz d'ammoniac libéré dans les gaz d'échappement, le procédé comprenant une étape de régénération de la matrice absorbante solide qui consiste en :
- la génération d'un gaz d'ammoniac de réapprovisionnement par décomposition chimique d'un précurseur d'ammoniac dans une unité de décomposition biochimique (3) installée à bord du véhicule et stockant au moins un composant protéique adaptée pour décomposer ledit précurseur d'ammoniac ;
- la séparation de l'eau du gaz d'ammoniac de réapprovisionnement au moyen d'une unité de séparateur liquide-vapeur ;
- l'orientation du gaz d'ammoniac de réapprovisionnement vers la matrice absorbante solide sur laquelle il est stocké.

2. Procédé SCR selon la revendication 1, dans lequel ledit composant protéique comprend au moins une enzyme.

3. Procédé SCR selon la revendication 2, dans lequel ladite enzyme est une uréase.

4. Procédé SCR selon l'une quelconque des revendications 1 à 3, dans lequel ladite unité de décomposition biochimique est équipée d'un dispositif de chauffage.

5. Procédé SCR selon l'une quelconque des revendications 1 à 4, comprenant une étape de compression du gaz d'ammoniac de réapprovisionnement au moyen d'une unité de mise sous pression de gaz, l'étape de compression étant effectuée avant l'étape d'orientation du gaz d'ammoniac de réapprovisionnement vers la matrice absorbante solide.

6. Procédé SCR selon l'une quelconque des revendications 1 à 5, dans lequel un flux du gaz d'ammoniac de réapprovisionnement est dosé dans les gaz d'échappement.

7. Procédé SCR selon l'une quelconque des revendications 1 à 6, dans lequel la matrice absorbante solide est stockée dans une première cuve (2) et le précurseur d'ammoniac est stocké dans une deuxième cuve (1), et dans lequel la deuxième cuve (1) est raccordée en communication avec ladite unité de décomposition biochimique (3), et ladite unité de décomposition biochimique (3) est raccordée en communication avec la première cuve (2).

8. Procédé SCR selon l'une quelconque des revendications 1 à 7, dans lequel la matrice absorbante solide comprenait pluralité de cellules contenant un ou plusieurs matériau(x) adapté(s) pour stocker l'ammoniac par sorption.

9. Procédé SCR selon l'une quelconque des revendications 1 à 8, dans lequel le précurseur d'ammoniac est un composé solide.

10. Procédé SCR selon l'une quelconque des revendications 1 à 8, dans lequel le précurseur d'ammoniac est une solution aqueuse d'urée.

11. Procédé SCR selon l'une quelconque des revendications 1 à 10, dans lequel la première cuve (2) dans laquelle la matrice absorbante solide est stockée est raccordée à une vanne de sécurité (8) configurée pour libérer l'excès d'ammoniac gazeux afin de détendre la pression au-dessus d'un point de consigne défini.

12. Système pour l'application d'un procédé SCR selon l'une quelconque des revendications précédentes, ledit système comprenant :
- une première cuve (2) installé à bord d'un véhicule et stockant au moins une matrice d'absorption solide sur laquelle de l'ammoniac est stocké par sorption,
- des moyens pour doser le gaz d'ammoniac libéré depuis la matrice absorbante solide, dans les gaz d'échappement, **caractérisé en ce que** le système comprend en outre
- des moyens pour orienter un flux de solution de précurseur d'ammoniac vers une unité de décomposition biochimique (3) installé à bord du véhicule et stockant au moins un composant protéique adapté pour décomposer ledit flux afin de générer un gaz d'ammoniac de réapprovisionnement ;
- une unité de séparateur liquide-vapeur (4) pour séparer l'eau du gaz d'ammoniac de réapprovisionnement ; des moyens pour orienter le gaz d'ammoniac de réapprovisionnement vers la matrice absorbante solide stockée dans la première cuve (2).

13. Système selon la revendication précédente, dans lequel ledit composant protéique comprend au moins une enzyme.

14. Système selon la revendication précédente, dans lequel ladite enzyme est une uréase.
